Europäisches Patentamt

European Patent Office

Office européen des brevets

(11) Publication number: **0 145 617**
**B1**

(12) **EUROPEAN PATENT SPECIFICATION**

(45) Date of publication of patent specification: **08.06.88**

(21) Application number: **84420185.5**

(22) Date of filing: **30.10.84**

(51) Int. Cl.⁴: **A 61 F 2/30,** A 61 F 2/32,
A 61 L 27/00

(54) Anisotropic surgical prosthesis.

(30) Priority: **04.11.83 US 548717**

(43) Date of publication of application:
**19.06.85 Bulletin 85/25**

(45) Publication of the grant of the patent:
**08.06.88 Bulletin 88/23**

(84) Designated Contracting States:
**DE FR GB IT**

(56) References cited:
**US-A-3 707 006**

(73) Proprietor: **Howmet Corporation**
**475 Steamboat Road**
**Greenwich, CT 06830 (US)**

(72) Inventor: **Carozza, Eugene J.**
**38 Jay Street**
**Succasunna New Jersey 07876 (US)**
Inventor: **Burd, Lamar**
**13 Smith Street**
**Andover New Jersey 07821 (US)**
Inventor: **Miller, Evan R.**
**Apt. 22D Village Green**
**Budd Lake New Jersey 07828 (US)**

(74) Representative: **Séraphin, Léon et al**
**PECHINEY 28, rue de Bonnel**
**F-69433 Lyon Cedex 3 (FR)**

Courier Press, Leamington Spa, England.

EP 0 145 617 B1

## Description

Technical Field

The invention relates to metal prosthetic devices disposed to replace portions of the skeletal system. More particularly, the invention relates to an improved femoral insert used in hip replacement surgery.

Background of the Invention

The replacement of a defctive hip joint with an implanted member is one of the significant recent advance of orthopedic surgery. Such implants are subjected to unique constraints. The materials used must be compatible with surrounding tissue, the materials must be strong and capable of being formed into relatively complex shapes, and, in addition, such members are subjected to significant mechanical loads that must be transmitted to the natural portions of the skeleton that interface with the prosthetic device. The replacement for the head of the femur normally has a stem that is implanted into the intermedullary cavity of the femur and is affixed by cement or by the growth of bone onto or into the face of the stem. In order to minimize the removal of material from the intermedullary cavity of the femur, the stem must be relatively slender. The loads on the stem, however, require that the material is strong.

A problem arises when a strong relatively slender metal member transmits applied loads to the surrounding bone. Some stress on the bone is necessary to prevent resorption, however; the load should be distributed as opposed to concentrated or local loading. Metal stems are normally formed of polycrystalline metal having a high elastic modulus and a flexural load applied to the stem deflects the stem significantly less than the surrounding bone. This can result in high local stresses and cause a detachment of the stem from the bone or the stem may actually fracture the surrounding bone.

To overcome all these constraints for repair or replace of bone structure in a living body, US—A—3 707 006 teaches composite orthopedic devices and methods which employ such devices. The orthopedic device comprises a substrate and a pyrolytic carbon coating on the substrate which pyrolytic carbon coating is physiologically compatible with living tissue and which has well defined physical properties with respect to bulk density, cristallite size, anisotrophy factor. Suitable substrates are those which have modulus of elasticity approximating that of natural bone such as polycristalline carbon, carbon fiber aggregates and refractory wire metals screens, so as to rather flex in harmony with the natural bone under applied stress, and thus to prevent a substantial concentration of stress at the prosthesis bone-interface.

One object of the present invention is to construct a prosthetic device compatible with its unique environment and having an elastic modulus that more closely matches that of the surrounding bone, while keeping sufficient strength.

An additional object of the invention is to provide a metal device that has an anisotropic elastic modulus with the minimum elastic modulus being aligned with the direction of maximum stress in the device such that strain on the bone surrounding the device is minimized.

A further object of the invention is to provide means for distributing the load applied by the prosthetic device to the surrounding bone to reduce high local stresss.

Other objects and advantags of the invention will be apparent from the description of the preferred embodiment or will be apparent from the practice of the invention.

Summary of the Invention

The invention is at first a metal prosthetic device comprising: a cast metal member disposed to replace a portion of the natural skeletal system, the elastic modulus of said metal member being anisotropic, the minimum elastic modulus of said metal member being aligned with the direction of maximum stress on said device, whereby the flexure of said device imparts minimum stress to the natural skeletal system interfaced with said device. Generally, the metal is of a cubic single crystal structure.

In a preferred embodiment, the device is a surgical prosthesis disposed to be implanted in the femur and to replace the head of the femur.

The present invention is also a method for casting a metal prosthetic device out of molten metal being capable of being formed into an anisotropic casting, said method utilizing means for shaping said casting, means for extracting heat from said molten metal within said shaping means, and means between said shaping means and said heat extracting means for orienting at least one crystallographic direction of said casting, said method comprising the steps of:

a) placing the molten metal within said shaping means;

b) controlling heat extraction from said molten metal such that heat flows primarily to said heat extracting member so as to form an anisotropic casting;

c) aligning a crystallographic direction in said casting with the direction in said device receiving maximum applied stress to reduce the elastic modulus of said casting in said direction.

Brief Description of the Drawings

Fig. 1 is a cross-sectional view of a femur having one embodiment of the invention implanted therein.

Fig. 2 is a cross-sectional view of one type of mold used to produce a single crystal hip prosthesis.

Fig. 3 is a cross-sectional view of another type of mold used to produce a single crystal hip prosthesis.

Fig. 4 is a cross-sectional view of still another type of mold used to produce a directionally solidified polycrystalline hip prosthesis.

## Description of the Preferred Embodiment

The invention concerns a prosthetic device that is disposed to replace a portion of the natural skeleton. The most commonly replaced portion of the human skeleton is the head of the femur and the acetabular joint in which it fits. It is the replacement for the head of the femur that is the principal application for the present invention and the disclosed embodiment is such a device. The invention, however, is broader than just a prosthetic replacement for the head of the femur and may be used in connection with other prosthetic devices that are implanted in bone and subjected to significant stress.

In accordance with the invention, the device is comprised of a cast metal member. The particular metal used must be compatible with its unique environment, and those skilled in the art are capable of selecting a metal or metal alloy suitable for implanting in such an application. In addition to the normal requirements of biocompatability and strength, the present invention requires that the metal used be capable of being cast in such a manner as to render its elastic modulus anisotropic. Preferably, this is accomplished by casting the metal to form a single crystal, but a directionally solidified casting having a predetermined grain shape and orientation may provide sufficient anisotrophy to provide the desired effect on the elastic modulus of the casting.

Preferably, the metal comprising the casting has a cubic crystallogrphic structure. One type of metal alloy which is known to be operable with the invention is the cobalt-chromium alloy, ASTM F 76, conventionally used in such prosthetic devices. For such a material it is possible to reduce the elastic modulus of a single crystal casting in a specific crystallographic direction by approximately 40%. For this particular alloy (which is typical of cubic metals) the direction of minimum elastic modulus is parallel to the edges of the cubic unit cell, i.e. the [001], [010] or [100] directions. The advantage of this reduction of the elastic modulus will be apparent from the description of the preferred embodiment.

Materials other than cubic metals also may be used with the present invention. Titanium and its alloys are known to be useful as a material for prosthetic devices; however, forming titanium as a single crystal that is stable at room temperature is difficult.

As noted above, it is preferred that the device be a single crystal. Not only can the anisotropy be controlled in such a structure but the lack of grain boundaries may allow the use of materials that would otherwise have undesirable properties due to grain bondary conditions. Use of single crystals also may allow the elimination of alloy constituents normally necessary to control grain boundary effects.

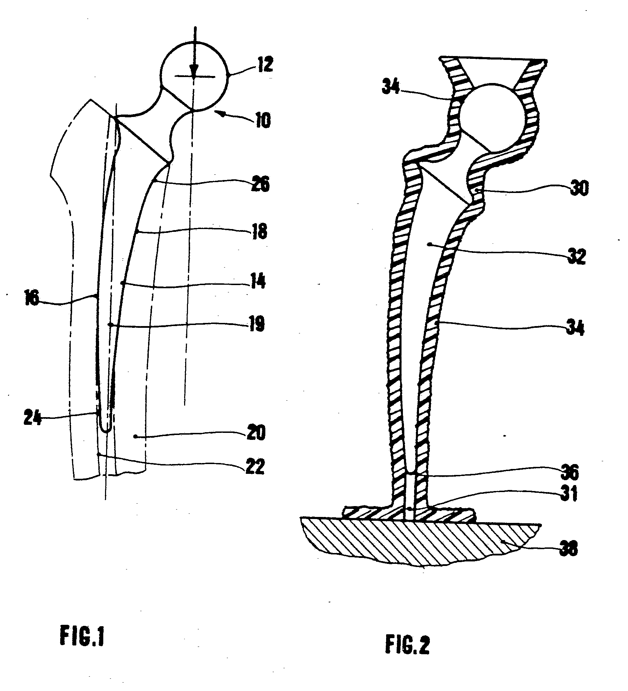

Fig. 1 depicts a surgical prosthesis disposed to replace the head of the femur and engage the acetabular joint in the pelvis. The device of Fig. 1, shown generally at 10, has an acetabular portion 12 and a femoral portion or stem 14. The device 10 is implanted in the head of the femur 20 in a conventional manner. The load transmitted to the femur 20 is applied through the acetabular portion 12 and the stem 14. As is apparent from Fig. 1, the applied load is not concident with the center of the femur 20 or the stem 14 so that a bending load is imposed on the stem.

As noted previously, the cross-sectional size of the stem is preferably small to reduce the amount of material that must be removed from the intermedullary cavity 22. In order for the stem 14 to withstand the relatively large imposed loads it must have a high elastic modulus. Yet when loads are applied through such a device, its elastic modulus is so much higher than that of the surrounding bone that shear stresses are set up at the prosthesis/bone interface. This can break the bond between the bone and the prosthesis. More drastic loading, either by higher loads or smaller stems, may actually cause rotation of the stem within the femur in response to the applied torque of the offset load and break the femur at the outer distal region 24, the inner proximal region 26 or both.

In the embodiment depicted, the load applied to the acetabular portion 12 creates a bending stress in the stem 14 in the direction of the arrow in Fig. 1. This creates maximum stress on the outer surface 16 and the inner surface 18 which are roughly parallel to the centerline 19 of the stem 14. Therefore, it is desired to reduce the elastic modulus in the direction of maximum applied stress, i.e. parallel to the centerline of the stem. In a cubic metal this is accomplished by aligning any of the edges of the unit cell of the cubic crystal with the centerline of the member under bending stress.

The alignment of certain crystallographic directions with the specific portion of the prosthetic device is accomplished by controlling the direction and rate of heat extraction during casting of the device. Metallic materials solididfy during casting when heat is removed from the melt by forming as a solid on existing solid material. If casting conditions are controlled, the melt can be made to soidify only on small solid particles of the melt material itself (rather than the mold or other solid particles in the melt), and, by limiting the number of such solid particles (nuclei) and controlling the direction of heat extraction from the melt, the melt can be solidified as a single crystal or a relatively small number of crystals aligned with the direction of heat extraction.

Cubic metals solidify at a higher rate along certain crystallographic directions, and this feature can be used to align such directions with the particular device being cast. In addition, metals can be made to solidify on an existing solid of the same metal in the same orientation as the solid. Therefore, by orienting the solid on which solidification is initiated, the crystallographic orientation of the casting may be oriented.

Fig. 2 depicts a mold 30 for casting a hip prosthesis using a small solid termed a "seed crystal" 31, to orient the crystallographic direction of the final casting. This embodiment is used to form a single crystal where all crystallographic directions are predetermined by the orientation of the seed crystal. The casting is shaped in the mold cavity 32 formed by a ceramic shell 34. The manner of producing such a mold is readily apparent to those skilled in the art of investment casting of metals. The mold 30 further includes a seed cavity 36 and a heat sink or "chill" 38. It is the function of the chill 38 to extract heat from the casting in contact with it thereby imparting a direction to the heat flow from the melt forming the casting. By manipulation of the temperature surrounding the ceramic shell 34, the temperature of the melt and the rate of heat extraction from the melt, the casting can be formed by solidification from the bottom up at a rate where the metal will only nucleate and grow as a single crystal.

To control the orientation of the single crystal being formed, the seed crystal 31 is placed within the seed cavity 36. The seed crystal has a known crystallographic orientation, and the orientation of the seed crystal within the seed cavity determines the orientation of the final casting. If, for example, the [001] direction of a cubic crystal is to be aligned with the centerline of the casting, that direction is oriented in the seed crystal to be parallel to the centerline. Upon initiation of the casting process the upper portion of the seed crystal is melted and the mold cavity filled. The extraction of heat from the melt through the seed crystal induces solidification at the seed/melt interface and the melt is solidified progressively from the seed crystal upward. If casting conditions are correct, the melt will solidify only in the orientation of the seed crystal as a single crystal. Those skilled in the art of casting metal single crystals can readily determine the casting conditions needed to produce an article in such a form.

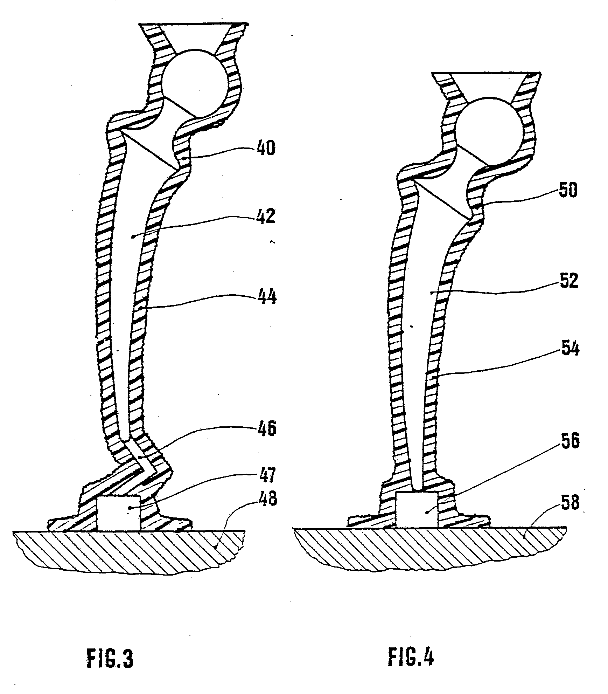

This method has an advantage over other methods of directionally solidifying metals because the orientation of the crystallographic directions of the casting can be controlled in all directions. This will be more clearly apparent when the alternative method of casting such a device, depicted in Fig. 3, is disclosed.

Fig. 3 depicts a mold 40 having a mold cavity 42 formed by a ceramic shell. The mold further includes a crystal selector 46, a growth cavity 47 in flow communication with the mold cavity 42 and a heat extracting member or chill 48.

When molten metal is poured into the mold cavity 42, it filled the selector 46 and the growth cavity 47 and solidification is initiated at the surface of the chill 48. While the orientation of the solidified metal upon the initial solidification is random, the rate of solidification in crystalline materials is not the same in all crystallographic directions. Thus, as the melt solidifies as a solid/liquid interface moving upward in the growth cavity 47, certain solid crystals having certain crystalline orientations will be favoured due to their more rapid rate of growth. The selector 46 will, because of its curvature, "select" from the advancing solid the crystal that is most rapidly advancing. At the exit of the selector 46, at the lower extremity of the mold cavity, the crystal being formed will have the crysalline direction that most rapidly solidifies aligned with the centerline of the mold.

In a cubic metal the crystallographic directions parallel to the edges of the cube are aligned with the direction of heat extraction. Therefore, these three directions are most favoured to be aligned with the centerline of the mold cavity at its lower extremity.

Unlike the seed crystal method, however, only one direction of the crystal can be controlled. In other words, if the [001] direction is parallel to the centerline of the mold, the [010] and [100] directions are mutually perpendicular but may be rotated at any orientation 360° around the [001] direction. By contrast, the seed crystal method controls the orientation of all crystallographic directions by orienting the seed crystal.

As noted above, the invention has been disclosed in terms of embodiments that are single crystals. One skilled in the art may use such teaching to produce an article having more than one crystal that nevertheless provides the benefits of the invention. For example, a polycrystalline member having the crystals or grains parallel to the direction of maximum stress with the orientation of the crystals aligned to reduce the elastic modulus in that direction would still be within the scope of the present invention.

Fig. 4 depicts a mold for making a directionally solidified casting. The mold 50 forms a mold cavity 52 utilizing a ceramic shell 54. The mold further includes a chill cavity 56 in contact with the chill 58. During the casting operation heat is directionally extracted from the melt such that solidification initiates at the chill surface within the chill cavity 56 with the liquid/solid interface moving upward. Appropriate control of heat flow, mold and melt temperatures and possible nucleation sites allows the formation of a polycrystalline casting having elongated grains aligned with the approximate centerline of the mold cavity 52.

Other embodiments of the invention will be apparent to those skilled in the art. The scope of the invention is not limited to the embodiments disclosed but is defined by the appended claims.

**Claims**

1. A metal prosthetic device comprising: a cast metal member (10) disposed to replace a portion of the natural skeletal system, the elastic modulus of said metal member (10) being anisotropic, the minimum elastic modulus of said metal metal (10) being aligned with the direction of maximum stress on said device, whereby the flexure of said device imparts minimum stress to the natural skeletal system interfaced with said device.

2. The device of Claim 1 wherein said device is a single crystal.

3. The device of Claim 2 wherein said metal comprises a cobalt-chromium alloy.

4. The device of Claim 2 wherein said crystal is cubic.

5. The device of Claim 4 wherein the single crystal has a crystal edge direction selected from the group consisting of [001], [010] and [100] generally aligned with the direction of maximum stress on said device.

6. The device of Claim 1 wherein said device is a surgical prosthesis disposed to be implanted in the femur.

7. The surgical prosthesis of Claim 6 adapted to replace the head of the femur, said prosthesis comprising: a femoral portion (14) and an acetabular portion (12), said femoral portion (14) being comprised of a single crystal of a cubic metal having a crystal direction selected from the group consisting of [001], [010] and [100] aligned to be parallel with the centerline of said femoral portion (14).

8. The surgical prosthesis of Claim 7 wherein said metal is an alloy designated as ASTM F 75.

9. A method for casting a metal prosthetic device out of molten metal being capable of being formed into an anisotropic casting, said method utilizing means (32; 42; 52) for shaping said casting, means (38; 48; 58) for extracting heat from said molten metal within said shaping means, and means (31; 46; 47; 56) between said shaping means (32; 42; 52) and said heat extracting means (38; 48; 58) for orienting at least one crystallographic direction of said casting, said method comprising the steps of:

a) placing molten metal within said shaping means (32; 42; 52);

b) controlling heat extraction from said molten metal such that heat flows primarily to said heat extracting member (38; 48; 58) so as to form an anisotropic casting;

c) aligning a crystallographic direction in said casting with the direction in said device receiving maximum applied stress to reduce the elastic modulus of said casting in said direction.

10. The method of Claim 9 including the step of placing a seed crystal (31) between said molten metal and said heat extracting member (38).

11. The method of Claim 9 including the step of selecting a single crystal forming in said molten metal and growing said single crystal in alignment with said crystallographic direction.

12. The method of Claim 9 wherein said casting is a single crystal.

13. The method of Claim 12 wherein said single crystal is a cubic metal.

14. The method of Claim 13 wherein said metal is a cobalt-chromium alloy.

15. The method of Claim 14 wherein said metal is ASTM F 75.

**Patentansprüche**

1. Metallprothese, umfassend: ein Metallgußstück (10), das zum Ersatz eines Teils des natürlichen Knochensystems angeordnet ist, wobei der Elastizitätsmodul des Metallgußstücks (10) anisotrop ist und der kleinste Elastizitätsmodul des Metallformstücks (19) mit der Richtung der größten auf die Prothese wirkenden Beanspruchung ausgerichtet ist, so daß ein Beugen der Prothese das an die Prothese angrenzende natürliche Knockensystem mit minimaler Beanspruchung beaufschlagt.

2. Metallprothese nach Anspruch 1, wobei die Prothese ein Einkristall ist.

3. Metallprothese nach Anspruch 2, wobei das Metall eine Cobalt-Chrom-Legierung umfaßt.

4. Metallprothese nach Anspruch 2, wobei der Kristall kubisch ist.

5. Metallprothese nach Anspruch 4, wobei der Einkristall eine Kirstallkanten-Richtung hat, die die [001]-, [010]- oder [100]-Richtung ist und im wesentlichen mit der Richtung der größten auf die Prothese wirkenden Beanspruchung ausgerichtet ist.

6. Metallprothese nach Anspruch 1, wobei die Prothese eine chirurgische Prothese ist, die in den Oberschnenkelknochen einzusetzen ist.

7. Chirurgische Prothese nach Anspruch 6, die den Obrschenkelkopf ersetzen soll, umfassend: einen femoralen Teil (14) und einen azetabulären Teil (12), wobei der femorale Teil (14) aus einem Einkristall eines kubischen Metalls mit einer [001]-, [010]- oder [100]- Kristallrichtung besteht, die mit der Mittenlinie des femoralen Teils (14) parallel ausgerichtet ist.

8. Chirurgische Prothese nach Anspruch 7, wobei das Metall eine mit ASTM F 75 bezeichnete Legierung ist.

9. Verfahren zum Gießen einer Metallprothese aus einer Metallschmelze, die zu einem anisotropischen Gußstück formbar ist, wobei das Verfahren Mittel (32; 42; 52) zum Formen des Gußstücks, Mittel (38; 48; 58) zur Wärmeabführung aus der Metallschmelze in den Formmitteln, und zwischen den Formmitteln (32; 42; 52) und den Wärmeabführmittel (38; 48; 58) befindliche Mittel (31; 46; 47; 56) zum Ausrichten wenigstens einer Kristallrichtung des Gußstücks verwendet, umfassend die folgenden Verfahrensschritte:

a) Einbringen von Metallschmelze in die Formmittel (32; 42; 52);

b) Regeln der Wärmeabführung aus der Metallschmelze derart, daß Wärme primär zu dem Wärmeabführelement (38; 48; 58) fließt, so daß ein anisoptropes Gußstück gebildet wird;

c) Ausrichten einer Kristallrichtung in dem Gußstück mit derjenigen Richtung in der Prothese, die de größten Beanspruchung ausgesetzt ist, um den Elastizitätsmodul des Gußstücks in dieser Richtung zu verringern.

10. Verfahren nach Anspruch 9, umfassend den Schritt: Einbringen eines Impfkristalls zwischen die Metallschmelze und das Wärmeabführelement (38).

11. Verfahren nach Ansrpuch 9, umfassend den Schritt: Auswählen eines sich in der Metallschmelze bild Einkristalls und Züchten diese Einkristalls in Ausrichtung mit der Kristallrichtung.

12. Verfahren nach Ansrpuch 9, wobei das Gußstück ein Einkristall ist.

13. Verfahren nach Anspruch 12, wobei der Einkristall ein kubisches Metall ist.

14. Verfahren nach Anspruch 13, wobei das Metall eine Cobalt-Chrom-Legierung.

15. Verfahren nach Anspruch 14, wobei das Metall ASTM F 75 ist.

## Revendications

1. Prothèse métallique constituée d'un élément en métal (10) moulé, destiné à remplacer une portion du squelette naturel le module d'élasticité dudit élément en métal (10) étant anisotrope at présentant sa valeur minimale dans la direction parallèle à la direction de la contrainte maximale subie par ladite prothèse, grâce à quoi la flexion de ladite prothèse ne communique qu'une contrainte minimale au squelette naturel qui se trouve en contact avec ladite prothèse.

2. Prothèse de la Revendication 1, dans laquelle ladite prothèse est un monocristal.

3. Prothèse de la Revendication 2, dans laquelle ledit métal est constitué d'un alliage cobalt-chrome.

4. Prothèse de la Revendication 2, dans laquelle ledit cristal est cubique.

5. Prothèse de la Revendication 4, dans laquelle le monocristal a l'une de des directions d'arête cristalline — choisie parmie les suivantes: [001], [010] et [100] — orientée (dans sa ligne génerale) parallèlement à la direction de la contrainte maximale subie part ladite prothèse.

6. Prothèse de la Revendication 1, dans laquelle ladite prothèse est une prothèse chirurgicale destinée à être implantée dans le fémur.

7. Prothèse chirurgicale de la Revendication 6, conçue pour remplacer la tête du fémur, ladite prothèse comportant une partie fémorale (14) et une partie acétabulaire (12), ladite partie fémorale (14) étant constituée d'un monocristal d'un métal cubique ayant l'une de ses directions cristallines — choisie parmi les suivantes: [001], [010] et [100] — orientée parallèlement à l'axe de ladite partie fémorale (14).

8. Prothèse chirurgicale de la Revendication 7, dans laquelle ledit métal est un alliage désigné sous le nom d'ASTM F 75.

9. Procédé permettant de couler une prothèse métallique à partir d'un métal liquide susceptible d'être façonné en une pièce moulée anisotrope, ledit procédé utilisant un dispositif (32, 42, 52) permettant de mettre en forme ladite pièce moulée, un dispositif (38, 48, 58) permettant d'extraire la chaleur dudit métal liquide à l'intérieure dudit dispositif de mise en forme et un dispositif (31, 46, 47, 56) — placé entre ledit dispositif de mise en forme (32, 42, 52) et ledit dispositif d'extraction de la chaleur (38, 48, 58) — permettant d'orienter au moins une direction cristallographique de ladite pièce moulée, ledit procédé comportant les étapes suivantes:

a) introduction du métal liquide dans ledit dispositif de mise en forme (32, 42, 52);

b) contrôle de l'extraction de la chaleur dudit métal liquide, de façon à ce que le flux de chaleur soit orienté essentiellement en direction dudit organe d'extraction de la chaleur (38, 48, 58), de manière à constituer une pièce molée anisotrope.

c) Orientation d'une direction cristallographique de ladite pièce moulée parallèlement à la direction de ladite prothèse qui subit la contrainte appliquée maximale, de manière à diminuer la module d'élasticité de ladite pièce moulée dans ladite direction.

10. Procédé de la Revendication 9, comportant l'étape consistant à placer un cristal-amorice (31) entre ledit métal liquide et ledit organe **) d'extraction de la chaleur (38).

11. Procédé de la Revendication 9, comportant l'étape constant à choisir un monocristal qui se forme dans ledit métal liquide et à faire croître ledit monocristal parallèlement à ladite direction cristallographique.

12. Procédé de la Revendication 9, dans lequel ladite pièce moulée est un monicristal.

13. Procédé de la Revendication 12, dans lequel ledit monocristal est un métal cubique.

14. Procédé de la Revendication 13, dans lequel ledit métal est un alliage cobalt-chrome.

15. Procédé de la Revendication 14, dans lequel ledit métal est l'alliage ASTM F 75.

FIG.1

FIG.2

FIG.3

FIG.4